(19) Europäisches Patentamt
European Patent Office
Office européen des brevets

(11) Veröffentlichungsnummer : **0 203 334**
**B1**

# (12) EUROPÄISCHE PATENTSCHRIFT

(45) Veröffentlichungstag der Patentschrift : 03.01.90

(51) Int. Cl.$^5$ : **G 01 N 33/84**

(21) Anmeldenummer : 86105035.9

(22) Anmeldetag : 12.04.86

## (54) Mittel und Verfahren zur Bestimmung von Calcium.

(30) Priorität : 24.04.85 DE 3514695

(43) Veröffentlichungstag der Anmeldung : 03.12.86 Patentblatt 86/49

(45) Bekanntmachung des Hinweises auf die Patenterteilung : 03.01.90 Patentblatt 90/01

(84) Benannte Vertragsstaaten : BE CH DE FR GB IT LI NL SE

(56) Entgegenhaltungen :
EP-A- 0 067 041
DE-A- 2 335 350
DE-A- 2 744 136
US-A- 3 822 116
ANALYST, Band 80, Oktober 1955, Seiten 713-729, London, GB; G. SCHWARZENBACH "The complexones and their analytical application"

(73) Patentinhaber : MERCK PATENT GESELLSCHAFT MIT BESCHRÄNKTER HAFTUNG
Frankfurter Strasse 250 Postfach 4119
D-6100 Darmstadt (DE)

(72) Erfinder : Wahl, Hans-Peter, Dr.
Lindenweg 22
D-6107 Reinheim (DE)
Erfinder : Würzburg, Uwe, Dr.
Dessauer Strasse 12
D-6110 Dieburg (DE)

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein Mittel und Verfahren zur Bestimmung von Calcium in Flüssigkeiten, insbesondere in Körperflüssigkeiten.

Die Bestimmung von Calcium in Flüssigkeiten unter Verwendung von Orthokresolphthalein-Komplexon ist in vielen technischen und klinischen Laboratorien eine übliche Methode. Die Bildung eines roten Komplexes mit Calcium wurde 1955 erstmals beschrieben (Analyst 80, 713 (1955)). Danach wurde die Methode laufend modifiziert, so daß sie in der Zwischenzeit auch an kontinuierlich messenden Systemen, Zentrifugal Analyzern und an diskret messenden Geräten durchgeführt werden kann.

Die Referenzmethode für die Bestimmung von Calcium ist die Atomabsorption, die jedoch sehr zeitaufwendig ist. Für die Bestimmung im Serum wird im klinischen Laboratorium heute im allgemeinen die Flammenphotometrie verwendet, die aber wegen der Störung durch Natriumionen eines geeigneten Flammenphotometers bedarf. Die photometrischen Bestimmungen eignen sich in der Regel nur für Calciumbestimmungen im Serum ; sie sind störanfällig und zeigen keine gute Übereinstimmung mit der Atomabsorption und der flammenphotometrischen Bestimmung. Ein weiterer Nachteil der bekannten photometrischen Bestimmungen ist darin zu sehen, daß Kaliumcyanid verwendet wird (DE-PS 2 335 350), woraus beim Ansäuern der tödlich giftige Cyanwasserstoff gebildet werden kann.

Aus dem US-Patent 3 822 116 ist ein Reagenz zur spektrophotometrischen Calciumbestimmung in Gegenwart eines zwitterionischen Puffers bekannt. Die dort genannten Puffersubstanzen und auch das bevorzugt verwendete Glycin haben in dem für die Farbreaktion nötigen pH-Bereich nicht die erforderliche Pufferkapazität, so daß die Empfindlichkeit dieser Methode erheblich herabgesetzt ist.

In der DE-OS 21 44 136 ist eine kolorimetrische Bestimmung von Calcium beschrieben, wobei ein spezielles Puffersystem aus einem Gemisch von Amidosulfonsäure, Dinatriumtetraborat und Alkalicarbonat verwendet wird. Dieses Puffersystem hat den Nachteil, daß eine Extinktionsmessung erst nach Wartezeiten bis zu 45 Minuten vorgenommen werden kann.

Der vorliegenden Erfindung liegt die Aufgabe zugrunde, ein Mittel und Verfahren zur photometrischen Bestimmung von Calciumionen zur Verfügung zu stellen, das einfach in der Ausführung, empfindlich und spezifisch ist und bei dem sowohl Serum, Plasma und Urin als auch z. B. Abwässer ohne Vorbehandlung direkt als Probe eingesetzt werden können. Dieses Mittel bzw. Verfahren sollte für die im Routinelabor verwendeten Photometer und Analysenautomaten verwendbar sein und hierfür in anwendungstechnisch praktikabler Form vorliegen. Eine weitere Aufgabe der vorliegenden Erfindung ist es, ein photometrisches Verfahren bzw. Mittel bereitzustellen, das kein Cyanid und keine giftigen Puffersubstanzen, wie Diethylamin und Diethanolamin enthält, da moderne Analysatoren eine Vielzahl von verschiedenen Tests gleichzeitig durchführen und so die Möglichkeit besteht, daß die Substanzen nach der Messung bzw. die Spülflüssigkeit mit Säuren aus anderen Tests zusammenkommen und so möglicherweise Cyanwasserstoff entsteht.

Die Aufgabe wurde erfindungsgemäß dadurch gelöst, daß ein Sulfonsäureamin als zwitterionischer Puffer verwendet wird. Überraschenderweise wurde gefunden, daß bei Verwendung dieses Puffers als Base die oben geschilderten Nachteile nicht auftreten.

Gegenstand der Erfindung ist ein Mittel zur Bestimmung von Calcium in Flüssigkeiten, enthaltend Orthokresolphthalein-Komplexon, eine Säure und einen alkalischen zwitterionischen Puffer, dadurch gekennzeichnet, daß der Puffer mindestens ein Sulfonsäureamin der allgemeinen Formel

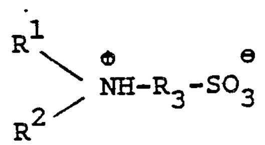

enthält,
worin

$R_1$ und $R_2$ gleich oder verschieden sind und H oder Alkyl, Hydroxyalkyl oder Cycloalkyl mit jeweils bis zu 8 C-Atomen und

$R_3$ Alkylen oder Hydroxyalkylen mit bis zu 8 C-Atomen bedeuten.

Ein weiterer Gegenstand der Erfindung ist ein Verfahren zur Bestimmung von Calcium in Flüssigkeiten durch Vermischen der zu untersuchenden Probe mit Orthokresolphthalein-Komplexon, einer Säure und einem zwitterionischen Puffer nach Anspruch 1, der eine Verbindung der allgemeinen Formel nach Anspruch 1 enthält und Messung der Intensität der gebildeten Farbe im pH-Bereich von 9 bis 11.

Bevorzugte Puffer sind solche der allgemeinen Formel, worin $R_1$ Cyclohexyl oder Hydroxyethyl, $R_2$ Wasserstoff und $R_3$ Alkyl oder Hydroxyalkyl mit 2 bis 6 C-Atomen bedeuten, insbesondere (3-(Cyclohexyl)-amino)propansulfonsäure, (2-(Cyclohexyl)-amino)ethansulfonsäure, (3-(Dihydroxyethyl)-amino)-2-hydroxypropansulfonsäure und (3-(Cyclohexyl)-amino)-2-hydroxypropansulfonsäure sowie Gemische dieser Verbindungen.

Das Mittel nach der Erfindung enthält Orthokresolphthalein-Komplexon, eine Säure, einen Puffer, gegebenfalls 8-Hydroxychinolin zur Maskierung von eventuell vorhandenen Magnesiumionen sowie Detergenzien und Stabilisatoren.

In einer bevorzugten Ausführungsform besteht das erfindungsgemäße Mittel aus einem sauren Farbreagenz, das Orthokresolsulfophthalein-Komplexon, 8-Hydroxychinolin und eine Säure enthält und einem alkalischen zwitterionischen Puffer. Beide Lösungen werden vor der Bestimmung so miteinander vermischt, daß der für die Farbreaktion notwendige pH-Wert im Bereich von etwa 9 bis 11 entsteht.

Das saure Farbreagenz enthält 0,01 bis 1 g/l Orthokresolphthalein-Komplexon, 0 bis 5 g/l 8-Hydroxychinolin und 0,01 bis 0,3 mol/l einer Säure, die mit den anderen Bestandteilen des Tests verträglich ist und die Farbreaktion nicht stört. Die Säure wird in einer Menge zugesetzt, daß ein pH-Wert im Bereich von 0,5 bis etwa 3 erzielt wird. Als Säuren können sowohl Mineralsäuren, wie Salzsäure, Schwefelsäure, Salpetersäure, Phosphorsäure, als auch feste, nicht flüchtige Säuren wie Kaliumhydrogensulfat, Sulfosalicylsäure, Naphthalinsulfonsäure, Naphthalindisulfonsäure, Salicylsäure, Acetylsalicylsäure, Citronensäure, Weinsäure, eingesetzt werden. Feste Säuren sind insbesondere dann erforderlich, wenn das Farbreagenz in lyophilisierter Form, als Tablette oder auf einem Träger gebunden zum Einsatz kommt. Das trocken zubereitete Farbreagenz wird durch Zugabe eines Lösungsmittels gebrauchsfertig gemacht. Das Lösungsmittel kann z. B. Wasser, die Probe selbst oder ein anderes Lösungsmittel sein.

Die zwitterionische Pufferlösung wird getrennt hergestellt. Sie enthält die Puffersubstanz in einer Konzentration von 0,05 bis 1 mol/l und gegebenfalls ein Netzmittel, wie Polyoxyethylenether oder Polyethylenglycolmonoalkylether. Die Pufferlösung wird mit einer Lauge, z. B. Natronlauge, auf den erforderlichen alkalischen pH-Wert eingestellt. Dieses Reagenz kann als Lösung eingesetzt werden; es kann auch als Lyophilisat, in Tablettenform oder an einem Träger, wie Papier, Glasfaser, Wolle, Kunststoffe, Kunstschäume usw. gebunden vorliegen und wird in diesem Fall durch Zugabe eines Lösungsmittels gebrauchsfertig gemacht. Das Lösungsmittel kann die zu bestimmende biologische Flüssigkeit oder ein anderes Lösungsmittel sein.

Für die Bestimmung von Calciumionen werden das Farbreagenz und der Puffer in geeigneter Form zusammengebracht, die Probe wird hinzugegeben und die Intensität des gebildeten Farbkomplexes bei einer Wellenlänge von 540 nm photometrisch bestimmt. Eine bevorzugte gebrauchsfertige Lösung enthält z. B. 0,02 bis 0,5 g/l Orthokresolphthalein-Komplexon, 0,5 bis 2,5 g/l 8-Hydroxychinolin, 0,01 bis 0,2 mol/l Salzsäure, 0,01 bis 5 g/l Polyoxyethylenether und 0,05 bis 1 mol/l des erfindungsgemäßen Puffers, der mit Natronlauge zuvor auf einen pH-Wert im Bereich von etwa 10 bis 11,8 gebracht worden ist.

## Beispiel 1

Es wurde folgende Calcium-Reagenzmischung hergestellt:

Farbreagenz:

| | |
|---|---|
| Orthokresolphthalein-Komplexon | 0,1 mmol/l |
| 8-Hydroxychinolin | 6,0 mmol/l |
| Salzsäure, pH 2 | 0,01 mol/l |

Puffer:

| | |
|---|---|
| (3-(Cyclohexyl)-amino) propansulfonsäure | 0,2 mol/l |
| Polyoxylethylenether | 2,0 g/l |
| NaOH, pH 10,7 | |

Die Reagenzien wurden 1 + 1 gemischt, wobei ein pH-Wert von 9,8 erreicht wurde.
Zur Durchführung der Bestimmung wurden folgende Mengen in Küvetten pipettiert:

| | Reagenzienleerwert | Standard | Probe |
|---|---|---|---|
| Wasser | 10 µl | - | - |
| Standard | - | 10 µl | - |
| Probe | - | - | 10 µl |
| Reagenzmischung | 1000 µl | 1000 µl | 1000 µl |

Die Lösungen wurden in der Küvette gemischt und die Extinktion wurde bei einer Wellenlänge von 540 nm bei 37 °C nach einer Minute abgelesen. Die Extinktion war mindestens 30 Min. stabil. Zur Ermittlung der Calciumionenkonzentration wurde die Reagenzienleerwertextinktion von der Extinktion des Standards und der Proben abgezogen. Die Calciumkonzentration berechnet sich demnach wie folgt:

$$\text{Calciumkonzentration} = \frac{\text{Extinktion Probe}}{\text{Extinktion Standard}} \times \text{Konzentration des Standards}$$

Ein Vergleich der nach dieser Methode ermittelten Calciumwerte mit den Werten einer flammenphotometrischen Messung zeigt einen streng linearen Zusammenhang, wie aus der folgenden Tabelle hervorgeht:

Tabelle I

| Flammenphotometrische Bestimmung Ca-Konzentration [mmol/l] | Photometrische Bestimmung Extinktion: 540 nm |
|---|---|
| 1,00 | 0,1330 |
| 2,01 | 0,2650 |
| 3,00 | 0,4000 |
| 4,02 | 0,5332 |
| 4,98 | 0,6650 |
| 10,00 | 1,3286 |

Beispiel 2

Es wurde folgende Calcium-Reagenzmischung hergestellt:
Farbreagenz:

| | |
|---|---|
| Orthokresolphthalein-Komplexon | 0,1 mmol/l |
| 8-Hydroxychinolin | 6,0 mmol/l |
| Kaliumhydrogensulfat | 0,06 mol/l |

Zwitterionischer Puffer:

| | |
|---|---|
| (3-(Cyclohexyl)-amino)-2-hydroxypropansulfonsäure | 0,2 mol/l |
| Polyethylenglycolmonoethylether | 2,0 g/l |
| NaOH, pH = 10,7 | |

Die Reagenzien wurden 1 + 1 gemischt, die Bestimmung erfolgte analog Beispiel 1.

Ein Vergleich der mit diesem Reagenz ermittelten Calciumwerte mit den Werten eines Reagenzes, das anstelle des erfindungsgemäßen Puffers Glycin enthielt, zeigt, daß die Empfindlichkeit des neuen Mittel wesentlich höher ist als mit dem bekannten Mittel.

Tabelle II

| Ca-Konzentration [mmol/l] | Puffer nach der Erfindung E = 540 nm | Glycin als Puffer E = 540 nm |
|---|---|---|
| 2 | 0,2640 | 0,0660 |
| 4 | 0,5278 | 0,1310 |
| 6 | 0,7924 | 0,1902 |
| 8 | 1,0560 | 0,2488 |
| 10 | 1,3203 | 0,3006 |

Beispiel 3

Aus Polyurethanschaum MA 5 044 der Firma Metzeler, Memingen, wurden Scheiben (Ø 18 mm, Höhe 8 mm) ausgebohrt. Diese wurden in einer 10 %igen Natriumcarbonat-Lösung 2 Stunden ausgekocht,

anschließend mit destilliertem Wasser mehrfach gespült und getrocknet. Zur Herstellung eines Träger-Testpacks wurden jeweils zwei dieser Scheiben mit folgender Farbreagenzlösung (200 µl) und zwitterionischer Pufferlösung (200 µl) getränkt.

Farbreagenz :

| | |
|---|---|
| Orthokresolphthalein-Komplexon | 0,5 mmol/l |
| 8-Hydroxychinolin | 30,0 mmol/l |
| Kaliumhydrogensulfat | 0,3 mmol/l |
| Zwitterionischer Puffer : | |
| (3-(Cyclohexyl)-amino)-2-hydroxypropansulfonsäure | 0,5 mol/l |
| Polyethylenglycolmonoethylether | 10,0 g/l |
| NaOH, pH = 10,7 | |

Die Scheiben wurden im Vakuum getrocknet. Zum Testansatz wurden je eine Scheibe mit Farbreagenz und eine Scheibe mit zwitterionischem Puffer in eine Einwegspritze (Ø 18 mm) eingelegt. Anschließend wurde 1 ml Wasser mehrmals in die so vorbereitete Einwegspritze eingesaugt, ausgedrückt und dann zusammen mit der Probe in eine Küvette gegeben. Messung und Auswertung erfolgte analog Beispiel 1. Es wurden die gleichen Ergebnisse erhalten wie in Beispiel 1.

## Patentansprüche

1. Mittel zur Bestimmung von Calcium in Flüssigkeiten, enthaltend Orthokresolphthalein-Komplexon, eine Säure und einen alkalischen zwitterionischen Puffer, dadurch gekennzeichnet, daß der Puffer mindestens ein Sulfonsäureamin der allgemeinen Formel

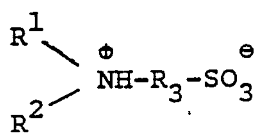

enthält,
worin
$R_1$ und $R_2$ gleich oder verschieden sind und H oder Alkyl, Hydroxyalkyl oder Cycloalkyl mit jeweils bis zu 8 C-Atomen und
$R_3$ Alkylen oder Hydroxyalkylen mit bis zu 8 C-Atomen bedeuten.

2. Mittel nach Anspruch 1, dadurch gekennzeichnet, daß es als Puffer (3-(Cyclohexyl)-amino)propansulfonsäure, (2-(Cyclohexyl)-amino)ethansulfonsäure, (3-(Dihydroxyethyl)-amino)-2-hydroxypropansulfonsäure und/oder (3-(Cyclohexyl)-amino)-2-hydroxypropansulfonsäure enthält.

3. Verfahren zur Bestimmung von Calcium in Flüssigkeiten durch Vermischen der zu untersuchenden Probe mit Orthokresolphthalein-Komplexon, einer Säure und einem zwitterionischen Puffer nach Anspruch 1, der eine Verbindung der allgemeinen Formel nach Anspruch 1 enthält und Messung der Intensität der gebildeten Farbe im pH-Bereich von 9 bis 11.

## Claims

1. Agent for the determination of calcium in liquids containing ortho-cresolphthalein complexone, an acid and an alkaline zwitterionic buffer, characterized in that the buffer contains at least one sulphonic acid amine of the general formula

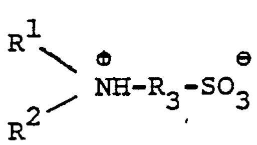

wherein
$R_1$ and $R_2$ are identical or different and denote H or alkyl, hydroxyalkyl or cycloalkyl having in each case up to 8 C atoms and
$R_3$ denotes alkylene or hydroxyalkylene having up to 8 C atoms.

2. Agent according to Claim 1, characterized in that it contains, as the buffer, (3-(cyclohexyl)-amino)-propanesulphonic acid, (2-(cyclohexyl)-amino)-ethanesulphonic acid, (3-(dihydroxyethyl)-amino)-2-hyd-

roxypropanesulphonic acid and/or (3-(cyclohexyl)-amino)-2-hydroxypropanesulphonic acid.

3. Process for the determination of calcium in liquids by mixing the sample to be examined with orthocresolphthalein complexone, an acid and a zwitterionic buffer according to Claim 1 which contains a compound of the general formula according to Claim 1, and measuring the intensity of the colour formed within the pH range from 9 to 11.

## Revendications

1. Moyen pour le dosage du calcium dans les liquides, renfermant orthocrésolphtaléine-complexone, un acide et un tampon zwitterionique alcalin, caractérisé en ce que le tampon renferme au moins une amine d'acide sulfonique de formule générale

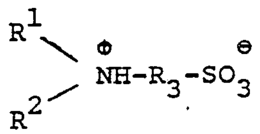

dans laquelle

$R_1$ et $R_2$ sont identiques ou différents et représentent H ou un alkyle, un hydroxyalkyle ou un cycloalkyle comportant chaque fois jusqu'à 8 atomes de C et

$R_3$ représente un alkylène ou un hydroxyalkylène comportant jusqu'à 8 atomes de C.

2. Moyen selon la revendication 1, caractérisé en ce qu'il renferme comme tampon de l'acide 3-cyclohexylaminopropanesulfonique, de l'acide 2-cyclohexyl-aminoéthanesulfonique, de l'acide 3-dihydroxyéthylamino-2-hydroxypropanesulfonique et/ou de l'acide 3-cyclohexylamino-2-hydroxypropanesulfonique.

3. Procédé de dosage du calcium dans les liquides, par mélange de l'échantillon à analyser avec orthocrésolphtaléine-complexone, un acide et un tampon zwitterionique, selon la revendication 1, ledit tampon renfermant un composé de la formule générale selon la revendication 1, et de détermination de l'intensité de la couleur formée dans l'intervalle de pH de 9 à 11.